# EUROPEAN PATENT APPLICATION

(11) **EP 1 468 708 A1**
(43) Date of publication of application: **20.10.2004**
(21) Application number: 03701133.5
(22) Date of filing: 21.01.2003
(51) Int. Cl.: A61M 37/00

(54) **ULTRASONIC PERCUTANEOUS PERMEATING DEVICE, ULTRASONIC PERCUTANEOUS PERMEATING KIT, AND ULTRASONIC PERCUTANEOUS PERMEATING METHOD**

(30) Priority: 21.01.2002 JP 2002012143
(71) Applicant: Matsushita Electric Works, Ltd., Kadoma-shi, Osaka-fu 571-8686 (JP)
(72) Inventor: MATSUMURA, Yuko, Kadoma-shi, Osaka 571-8686 (JP); SATO, Yasuhiro, Kadoma-shi, Osaka 571-8686 (JP)
(74) Representative: Dallmeyer, Georg, Dipl.-Ing.
(86) International application number: PCT/JP2003/000455
(87) International publication number: WO 2003/061753

(57) **Abstract**

Upon allowing a medicine 1 containing an active ingredient to penetrate an organism 2 from a skin surface 2a, this ultrasonic percutaneous penetration device A allows vibration of ultrasonic waves to penetrate the organism from the skin surface. This device is provided with an irradiation unit 4 that applies ultrasonic waves having a frequency of not less than 0.5 MHz from skin 2a or a surface capable of contacting the medicine, and a control unit 3 that controls irradiation conditions of the irradiation unit.

## Description

### TECHNICAL FIELD

The present invention relates to an ultrasonic percutaneous penetration device which allows a medicine containing an active ingredient to penetrate a portion of the organism percutaneously by using ultrasonic vibration.

### BACKGROUND ART

Conventionally, a DDS (drug delivery system, percutaneous medicine delivery system) has been developed as a technique which allows a medicine to penetrate percutaneously so that an active ingredient of the medicine is applied to the skin and circulatory system, and an iontophoresis method which allows penetration by using electricity has developed considerably as a method of the chemical administration. Moreover, techniques using ultrasonic vibration have been gradually developed, and, for example, Japanese Laid-Open Patent Publication No. 52-115591 has proposed a method in which a medicine is allowed to penetrate a skin through irradiation of ultrasonic waves having a frequency of 1MHz for the treatment of herpes, and Japanese Patent No. 2710281 has proposed a method in which the ultrasonic wave output is properly controlled so as to allow a medicine to penetrate a circulatory system.

### DISCLOSURE OF INVENTION

In the above-mentioned method disclosed in Japanese Laid-open Patent Publication No. 52-115591, in an attempt to achieve the osmotic effect of a medicine for the skin lesion, the medicine is allowed to penetrate by using an ultrasonic wave having a frequency of 1 MHz with 1 W/cm². However, when action of ultrasonic waves to a target portion such as skin and fat and characteristics of the ultrasonic waves are taken into consideration, it cannot be said that the above-mentioned irradiation conditions of ultrasonic wave are appropriate.

Moreover, in the method disclosed in Japanese Patent No. 2710281, the target portion for penetration of the medicine is the circulatory system, and the ultrasonic waves are controlled so as to allow the medicine to penetrate more effectively. However, the objective of the invention described in this patent gazette is limited to penetration of a medicine to only the circulatory system, and no description has been given to the action thereof to the skin that forms a via site.

Moreover, neither of the above-mentioned patent gazettes describes anything about timing between the irradiation of ultrasonic waves and the application of a medicine to the skin. Here, when the fact that even after irradiation of ultrasonic waves, the penetration effects of the ultrasonic waves continue, which has been newly discovered by the inventors, etc. of the present invention, is taken into consideration, the irradiation of ultrasonic waves and the application of a medicine to the skin need not be carried out simultaneously.

An objective of the present invention is to provide an ultrasonic percutaneous penetration device which allows a medicine to effectively penetrate the skin, fatty tissue and muscle tissue safely.

Upon allowing the medicine containing an active ingredient to penetrate the organism from the skin surface, the ultrasonic percutaneous penetration device allows vibration of ultrasonic waves to penetrate the organism from the skin surface, and is provided with:
an irradiation unit that applies ultrasonic waves having a frequency of not less than 0.5 MHz from skin or a surface capable of contacting the medicine; and
a control unit that controls irradiation conditions of the irradiation unit.

In accordance with the ultrasonic percutaneous penetration device of the present invention, the ultrasonic waves having a frequency of not less than 0.5 MHz are applied percutaneously. By utilizing two characteristics, that is, a characteristic of ultrasonic waves for loosening intercellular lipid of the skin corneum and a characteristic of giving different actions to the organism depending on different frequencies, it is possible to allow a medicine to effectively penetrate a desired portion for penetration. For this reason, it is possible to obtain effects of improving the penetrating property several times higher than a normal case in which the agent is simply applied to the skin. Moreover, it is possible to prevent occurrence of skin disorder such as bum on the skin surface, and consequently to allow a medicine to effectively penetrate the organism safely.

Here, the control unit controls at least one of factors including the frequency, irradiation power, period between on and off of power and irradiation time, which are irradiation conditions of ultrasonic waves.

Thus, it is possible to prepare optimal irradiation conditions in response to a portion for penetration and a medicine to be applied, and consequently to allow a medicine to effectively penetrate the organism safely.

Moreover, a detection unit, which detects the depth of a portion for penetration of the medicine, may be prepared, and in this case, the control unit controls the irradiation conditions so as to allow the medicine to penetrate to the depth detected by the detection unit.

Thus, it becomes possible to allow the medicine to positively penetrate a desired portion.

Here, the irradiation unit may apply two or more ultrasonic waves having different frequencies. Moreover, the irradiation unit may apply an ultrasonic wave having a frequency of virtually 1 MHz and an ultrasonic wave having a frequency of not less than 2 MHz.

By applying two or more ultrasonic waves having different frequencies to the skin, different actions are exerted on the organism by the respective ultrasonic waves so that it becomes possible to allow the medicine to more effectively penetrate a desired portion for penetration.

Moreover, the above-mentioned device may further include any one of the following tools: a thermal tool for warming a portion to be subjected to penetration of the medicine, a massaging tool for repeatedly pressing and releasing the portion to be subjected to penetration of the medicine, an electrostimulator that applies electrical stimulation to the portion to be subjected to penetration of the medicine and a photostimulator that applies photic stimulation to the portion to be subjected to penetration of the medicine.

Upon allowing the medicine containing an active ingredient to penetrate the organism from the skin surface, the ultrasonic percutaneous penetration kit of the present invention allows vibration of ultrasonic waves to penetrate the organism from the skin surface, and is provided with:
a medicine containing an active ingredient;
an irradiation unit that applies ultrasonic waves having a frequency of not less than 0.5 MHz from a surface capable of contacting the medicine; and
a control unit that controls irradiation conditions of the irradiation unit.

Here, the above-mentioned active ingredient may be a whitening component, and in this case, the control unit controls the frequency of the ultrasonic wave to a frequency in a range from 3 to 7 MHz.

By applying the ultrasonic wave to the skin under the above-mentioned conditions, the medicine is allowed to safely penetrate the base layer of the skin, and also to effectively react with melamine existing in the base layer; thus, it becomes possible to obtain high whitening effects.

Moreover, the whitening component may be at least one member selected from the group consisting of vitamin C, vitamin C derivatives, kojic acid, glucoside, glutathione, kiwifruit extract, rose fruit extract, arbutin and acerola extract, and the formulation may be at least one type selected from the group consisting of gel type, lotion type, liquid type and impregnated type.

With respect to the above-mentioned active ingredient, at least one component for treating wrinkles, selected from the group consisting of vitamin A, vitamin A acid derivatives, retinol, glutathione, α-hydroxy acid and a cell activation agent, may be used.

Moreover, with respect to the above-mentioned active agent, at least one active ingredient used for burning fat, selected from the group consisting of vitamin B group, capsaicin and caffeine, may be used. In the above-mentioned case, the frequency of ultrasonic wave is controlled to not less than 0.7 MHz by the above-mentioned control unit.

By applying ultrasonic waves to the skin under the above-mentioned conditions, it is possible to effectively burn fat, and consequently to prepare appropriate slimming effects.

Furthermore, with respect to the above-mentioned active ingredient, at least one active ingredient used for trichophytosis treatment, selected from the group consisting of a thiocarbamate-based agent, imidazole-based agents, an allylamine-based agent, an amorolfine-based agent, undecylenic acid and derivatives thereof, an antifungal agent and an antitrichophyton agent, which are effective for Trichophyton located in a deep portion of the corneum layer, may be used.

Here, the above-mentioned medicine may be impregnated into a base material.

By impregnating the agent into the base material, the agent is made to work on the skin surface stably for a long time; thus, the penetrating effects of the medicine can be improved, and the medicine such as a liquid can be easily handled.

In the ultrasonic percutaneous penetration method in accordance with the present invention, simultaneously as the medicine containing an active ingredient is made to contact the skin, ultrasonic waves having a frequency of not less than 0.5 MHz are applied to the skin surface through the above-mentioned medicine.

In another method, after making the medicine containing an active ingredient in contact with the skin, ultrasonic waves having a frequency of not less than 0.5 MHz are applied to the skin surface through a medium that transmits the ultrasonic waves.

As described above, the process in which the medicine is made in contact with the skin is carried out prior to application of the ultrasonic waves; therefore, the medicine is not susceptible to limitations in the shape or the like required to transmit ultrasonic waves to the skin. Therefore, the medicine to be penetrated can be used as a packed product and the like.

In still another method, after having applied ultrasonic waves having a frequency of not less than 0.5 MHz to the skin surface, a medicine containing an active ingredient is made in contact with the skin to which the ultrasonic waves have penetrated.

Moreover, in still another method, two or more processes are selected from the following three processes: a process in which a medicine containing an active ingredient is made in contact with the skin; a process in which ultrasonic waves having a frequency of not less than 0.5 MHz are applied to the skin surface; and a process in which, simultaneously as the medicine containing an active ingredient is made in contact with the skin, ultrasonic waves having a frequency of not less than 0.5 MHz are applied to the skin surface through the medicine, and the selected processes are carried out time-serially in succession.

### BRIEF DESCRIPTION OF DRAWINGS

Fig. 1 is a schematic drawing that shows a structure of an ultrasonic percutaneous penetration device in accordance with an embodiment of the present invention.
Figs. 2A to 2C are schematic drawings that show a time-serial sequence of processes in one example of an ultrasonic percutaneous penetration method in which the ultrasonic percutaneous penetration device of the embodiment of the present invention is used.
Figs. 3A to 3C are schematic drawings that show a time-serial sequence of processes in another example of an ultrasonic percutaneous penetration method in which the ultrasonic percutaneous penetration device of the embodiment of the present invention is used;
Fig. 4 is a schematic drawing that shows a structure of another example of an ultrasonic percutaneous penetration device in accordance with the embodiment of the present invention;
Fig. 5 is a graph that shows comparisons in penetration quantity of vitamin C in specific example 1 of the present invention;
Fig. 6 is a graph that shows continuous penetration effects of a medicine after irradiation with ultrasonic waves in specific example 2 of the present invention; and
Fig. 7 is a graph that shows comparisons in changes in the color of pigmentation in specific example 3 of the present invention.

### BEST MODE FOR CARRYING OUT THE INVENTION

The following description discusses embodiments of the present invention.

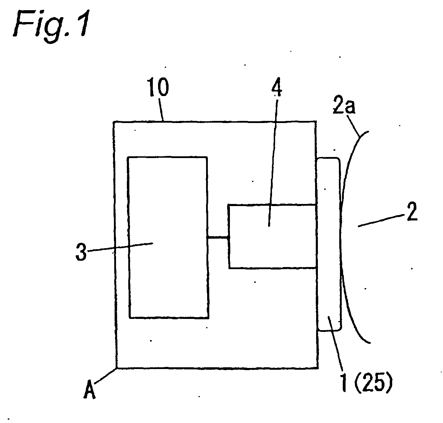

Fig. 1 shows an example of an ultrasonic percutaneous penetration device A of the present invention. This ultrasonic percutaneous penetration device A is provided with a control unit 3 and an irradiation unit 4 placed inside a casing 10. This irradiation unit 4 is provided with an ultrasonic vibrator that is used for generating ultrasonic waves to be applied to a skin surface 2a. Moreover, the control unit 3 has an electric circuit that controls irradiation conditions of ultrasonic waves to be applied from the ultrasonic transducer in the irradiation unit 4. The control unit 3 controls at least one of irradiation conditions of ultrasonic waves including frequency, irradiation power, period between on and off of output (duty ratio) and irradiation time. Here, the controlling operations of irradiation conditions by the control unit 3 may be automatically carried out or may be manually carried out. Further, the irradiation unit 4 of the ultrasonic percutaneous penetration device A is placed close to the skin surface 2a of an organism 2 so that ultrasonic waves are applied from the ultrasonic transducer of the irradiation unit 4 to the skin surface 2a, and then allowed to penetrate the organism 2 from the skin surface 2a. In this case, the ultrasonic waves are preferably applied to the skin surface 2a located on the surface side of a target portion of the organism 2 that the ultrasonic waves penetrate. The adjustments of the irradiation position, the start of the irradiation and the like by the irradiation unit 4 may be automatically carried out, or may be manually carried out.

By applying ultrasonic waves generated by the ultrasonic percutaneous penetration device A of the present invention to the skin surface 2a of the organism 2, vibration of the ultrasonic waves is allowed to locally penetrate a target portion of the organism 2 through the skin surface 2a. Thus, intercellular lipid of the skin corneum is loosened by the vibration of the ultrasonic waves (a state in which the fluidity between cells becomes higher), the penetrating property of a medicine 1 and its active ingredient to the target portion of the organism 2 is increased (the quantity of penetration of the medicine 1 and its active ingredient is increased). Moreover, ultrasonic waves are generated under conditions suitable for allowing the medicine 1 to penetrate the target portion of the organism 2, and applied to the skin surface 2a so that it becomes possible to further improve the penetrating property of the medicine 1 and its active ingredient to the target portion of the organism 2.

Here, the penetrating capability of ultrasonic waves to the organism 2 differs depending on the frequency thereof, and when the frequency is high, the energy is consumed at a shallow portion (in the vicinity of the skin) of the organism 2, with the result that the energy fails to penetrate the organism 2 into a deep portion. In contrast, when the frequency is low, the consumption of the energy at a shallow portion is small, with the result that the energy is allowed to penetrate the organism 2 into a deep portion. In accordance with the ultrasonic percutaneous penetration device A of the present invention, by utilizing the above-mentioned characteristics of ultrasonic waves, the medicine 1 is allowed to locally penetrate a target portion of the organism 2 effectively.

Upon application of ultrasonic waves from the ultrasonic percutaneous penetration device A of the present invention, the irradiation conditions thereof can be altered on demand depending on the target effects, and in an attempt to achieve whitening effects, melamine located in the skin base layer that forms a shallow portion of the organism 2 is given as a target to which the active ingredient is applied; therefore, since the target is in a shallow portion from the skin surface 2a, the medicine 1 is allowed to effectively penetrate by using ultrasonic waves having a high frequency (3 to 7 MHz). Moreover, in an attempt to reduce wrinkles, the skin corium forms a target to which the active ingredient is applied; therefore, the medicine 1 is allowed to effectively penetrate by using ultrasonic waves having an intermediate frequency (1 to 3 MHz). Furthermore, in an attempt to achieve slimming effects, fat tissues and muscle layers, located at deeper portions in the organism 2, form a target to which the active ingredient is applied; therefore, the medicine 1 is allowed to effectively penetrate by using ultrasonic waves having a low frequency (0.5 to 2 MHz, preferably, 0.7 to 1 MHz) into a deep portion thereof.

In this manner, the irradiation conditions of ultrasonic waves, which are suitable for the depth of the target portion for penetration of the medicine 1, need to be used in order to allow the medicine 1 to effectively penetrate. Therefore, the ultrasonic percutaneous penetration device A is capable of generating ultrasonic waves having a frequency from 0.5 to 5 MHz, and the frequency of the ultrasonic waves can be appropriately set within this range.

The above description has discussed the frequency conditions of ultrasonic waves to be used for effectively penetrating the medicine 1, and conditions of the irradiation power of ultrasonic waves also need to be taken into consideration. As described above, in the case of high frequency of ultrasonic waves, since energy is consumed in the shallow portion, the quantity of heat generation becomes greater at the corresponding portion. Moreover, in the case when the irradiation power of ultrasonic waves is great, since the quantity of heat generation also becomes greater, there is the possibility of generation of skin disorder such as bum. For this reason, it is essential to use irradiation power in which the skin disorder does not arise. In order to achieve high penetration effects of the medicine 1 and its active ingredient without causing the skin disorder, the irradiation condition of ultrasonic waves is preferably set to not more than 2 W/cm², more preferably, not more than 0.7 W/cm², with respect to the skin surface 2a, within the above-mentioned frequency range. Thus, it becomes possible to prevent the energy of ultrasonic waves from concentrating on the skin, and consequently to allow the medicine 1 and its active ingredient to effectively penetrate safely. Here, when the penetrating property of the agent 1 is taken into consideration, the irradiation power is preferably set to not less than 0.2 W/cm² with respect to the skin surface 2a.

With respect to the medicine 1 to be percutaneously penetrated by using the present invention, which contains an active ingredient, at least one product, selected from the group consisting of those compositions prepared as cosmetics (skin lotion, milky lotion, essence, cream, gel-state cosmetics, etc.), medical cosmetics, medicine articles and quasi-drugs as well as those compositions that are water-soluble or oil-soluble with high fluidity, and prepared on demand, may be used. Moreover, with respect to the formation of the medicine 1 that corresponds the type thereof, at least one type, selected from the group consisting of gel type, lotion type, liquid type and impregnated type, may be used.

The active ingredient to be contained in the medicine 1 can be appropriately selected depending on the target effect. With respect to the medicine 1 used for obtaining whitening effects, a whitening agent is used, and this whitening agent contains at least one active ingredient (whitening component) selected from the group consisting of vitamin C, vitamin C derivatives, kojic acid, glucoside, glutathione, kiwifruit extract, rose fruit extract, arbutin and acerola extract. In an attempt to reduce wrinkles, the medicine 1 preferably contains at least one active ingredient selected from the group consisting of vitamin A, vitamin A acid (retinoic acid) derivatives, retinol, glutathione, α-hydroxy acid and a cell activation agent. Moreover, in an attempt to provide a slimming effect, the medicine 1 preferably contains at least one active ingredient used for burning fat, selected from the group consisting of vitamin B group, capsaicin and caffeine. Moreover, in an attempt to provide effects for trichophytosis treatment, the medicine 1 preferably contains at least one active ingredient selected from the group consisting of a thiocarbamate-based agent, an imidazole-based agent, an allylamine-based agent, an amorolfine-based agent, undecylenic acid and derivatives thereof, an antifungal agent and an antitrichophyton agent, which are effective for Trichophyton located in a deep portion of the corneum layer.

Upon applying ultrasonic waves to the organism 2 while allowing the medicine 1 to penetrate the target portion of the organism 2 by using the ultrasonic percutaneous penetration device A, the following three methods are proposed: A first method has an arrangement in which the medicine 1. and ultrasonic waves are simultaneously applied to the organism 2. As shown in Fig. 1, in this method, the medicine 1 is applied onto the skin surface 2a through a process such as coating, and with the medicine 1 being left on the skin surface 2a, ultrasonic waves are applied to the skin surface 2a of the organism 2. In this case, since the medicine 1 is allowed to penetrate from the skin surface 2a while ultrasonic waves are being applied to the organism 2, the penetrating effect of the medicine 1 becomes higher in comparison with the case in which no ultrasonic waves are used.

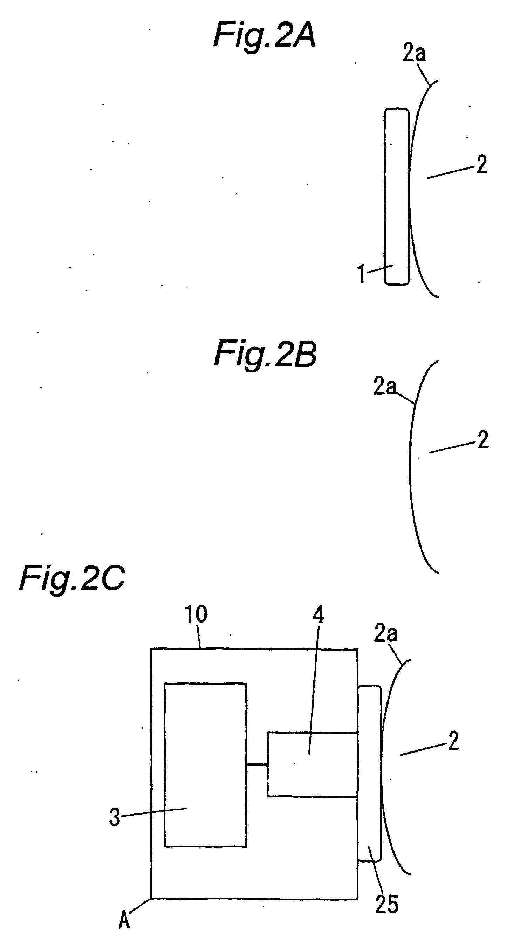

A second method is a method in which after the application of the medicine 1, ultrasonic waves are applied thereto. In this method, as shown in Fig. 2A, after the application of the medicine 1 onto the skin surface 2a through a process such as coating, the medicine 1 is left or removed therefrom to hardly remain on the skin surface 2a as shown in Fig. 2B, and as shown in Fig. 2C, ultrasonic waves are then applied to the skin surface 2a of the organism 2. In this case, after a lapse of several minutes (about 5 to 10 minutes) from the time at which the medicine 1 has been virtually eliminated from the skin surface 2a, ultrasonic waves are applied thereto; however, the residual medicine 1 and active ingredient on the skin surface 2a are allowed to percutaneously penetrate so that the penetrating effect of the medicine 1 becomes higher in comparison with the case in which no ultrasonic waves are used.

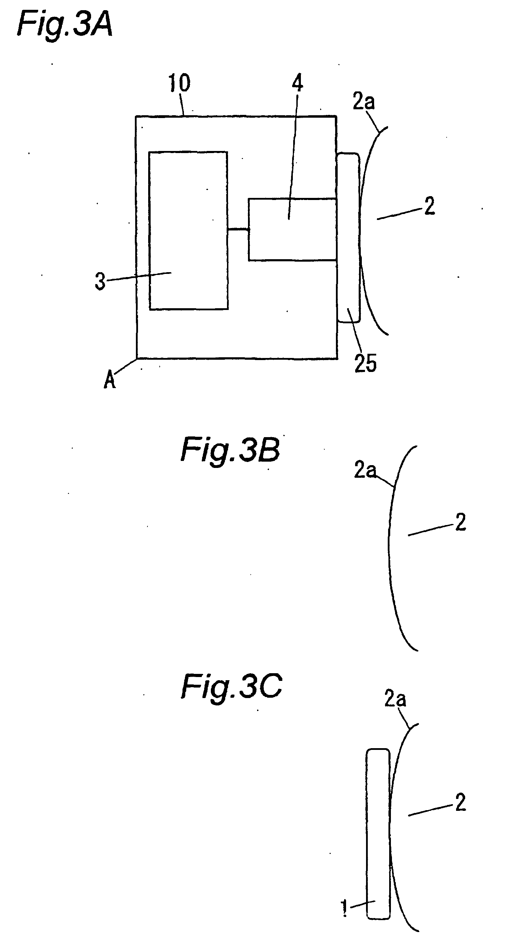

A third method is a method in which ultrasonic waves are applied prior to application of the medicine 1. In this method, as shown in Fig. 3A, after ultrasonic waves have been applied onto the skin surface 2a of the organism 2, the ultrasonic percutaneous penetration device A and an ultrasonic wave transmission medium 25, which will be described later, are removed, as shown in Fig. 3B, and as shown in Fig. 3C, the medicine 1 is applied onto the skin surface 2a through a coating process and the like. In this case, the medicine 1 is used after the application of the ultrasonic waves; however, since the loosened state of intercellular lipid of the skin corneum due to the irradiation with ultrasonic waves continues for at least 30 minutes, the medicine 1 is applied to the skin surface 2a through a coating process or the like within 30 minutes since the application of ultrasonic waves so that the medicine 1 and its active ingredient are allowed to percutaneously penetrate so that the penetrating effect of the medicine 1 becomes higher in comparison with the case in which no ultrasonic waves are used.

With respect to the above-mentioned three methods, two or more methods may be combined together to be carried out time-serially in succession, or two or more methods may be successively repeated.

Upon application of ultrasonic waves to the skin surface 2a by using the ultrasonic percutaneous penetration device A, the outer surface of the irradiation unit 4 or the outer surface of the casing 10 may be directly made in contact with the skin surface 2a, or, as shown in Figs. 2 and 3, the ultrasonic wave transmission medium 25 may be interpolated between the irradiation unit 4 and the skin surface 2a. The ultrasonic wave transmission medium 25, which is used for transmitting ultrasonic waves generated by the irradiation unit 4 to the skin surface 2a, may be made from a gel-state aqueous solution in which, for example, carboxy methyl cellulose (CMC) is blended. The ultrasonic wave transmission medium 25, which is made closely in contact with both of the irradiation unit 4 and the skin surface 2a upon application of ultrasonic waves, is applied onto the skin surface 2a or impregnated into a base material, which will be described later, so that the resulting base material is bonded to the skin surface 2a; thus, this is interpolated between the irradiation unit 4 and the skin surface 2a. Moreover, the ultrasonic wave transmission medium 25 may contain the above-mentioned active ingredient to be prepared as the medicine 1. Furthermore, the ultrasonic wave transmission medium 25, which contains the above-mentioned medicine 1, may be used.

Moreover, in the case when the medicine 1 is used for the organism 2, in addition to applying the medicine 1 to the skin surface 2a, the medicine 1 may be impregnated into a base material to be maintained therein so that the resulting base material is bonded to the skin surface 2a. With respect to the base material, a cloth such as nonwoven fabric and a sheet-shaped material such as paper, which are easily available, may be used. With this arrangement in which the base material impregnated with the medicine 1 to be maintained therein is bonded to the skin surface 2a, it is possible to maintain the state in which the medicine 1 is made closely in contact with the skin surface 2a for a long time, and consequently to improve the penetration effects of the medicine 1; moreover, it becomes possible to prevent the medicine 1 from dripping down, and consequently to achieve easiness in handling.

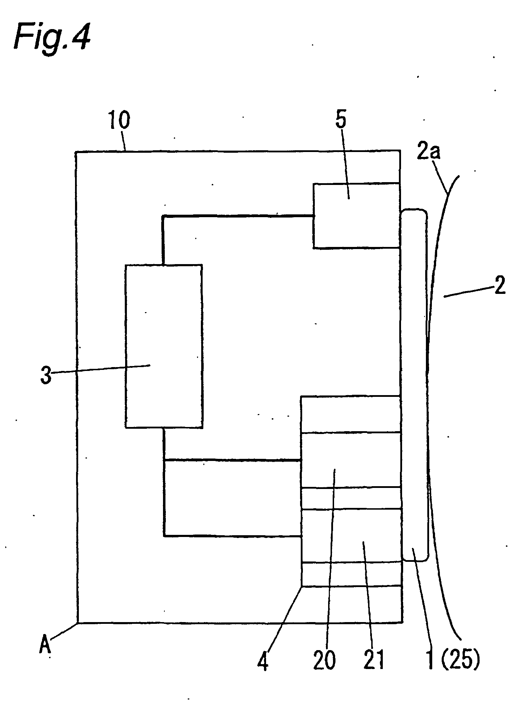

Fig. 4 shows another embodiment. This ultrasonic percutaneous penetration device A is provided with a detection unit 5 which detects the depth of a portion that is subjected to penetration of the medicine 1. The detection unit 5 utilizes the characteristic of ultrasonic waves for use in diagnosis. While applying ultrasonic waves to the organism 2, this detection unit 5 receives the ultrasonic waves reflected from the organism 2 so that the depth of a portion to be subjected to the penetration of the medicine 1 is measured and detected. The results of detection by the detection unit 5 are sent to the control unit 3 in which irradiation conditions of ultrasonic waves that are suitable for the depth detected by the detection unit 5 are determined. Then, based upon the irradiation conditions determined by the control unit 3, the irradiation of ultrasonic waves from the ultrasonic transducer in the irradiation unit 4 is controlled.

Moreover, two ultrasonic transducers 20 and 21 are installed in the irradiation unit 4 of the ultrasonic percutaneous penetration device A. These ultrasonic transducers 20 and 21 are different in their types, that is, different in frequencies in ultrasonic waves to be generated or different in their periods between on and off outputs. Here, these ultrasonic vibrators 20 and 21 may be respectively used individually or may be used alternately, depending on purposes, portions to be used or irradiation conditions. The other structures and the methods of use are the same as those described in the above-mentioned embodiments. Additionally, not limited to two, three or more ultrasonic vibrators may be installed. With this arrangement, ultrasonic waves can be simultaneously applied to two or more portions of the organism 2, or two or more kinds of ultrasonic waves having different conditions may be applied alternately.

In the present embodiment, based upon the detection by the detection unit 5, the depth of a portion to be subjected to penetration of the medicine 1 is accurately confirmed, and the irradiation conditions of ultrasonic waves suitable for the depth can be selected so that it becomes possible to allow the medicine 1 effectively penetrate the target portion.

Moreover, the ultrasonic percutaneous penetration device A of the present invention may be provided with a control unit 3 and an irradiation unit 4 that can generate two or more ultrasonic waves having different frequencies (frequency bands). For example, in the case when two ultrasonic waves having different frequencies are generated, the ultrasonic percutaneous penetration device A shown in Fig. 4 allows the control unit 3 to carry out controlling operations so that the frequency of an ultrasonic wave to be generated from the ultrasonic transducer 20 and applied to the skin surface 2a is made different from the frequency of an ultrasonic wave to be generated from the ultrasonic transducer 21 and applied to the skin surface 2a. In the case when these two ultrasonic waves having mutually different frequencies are generated, with respect to the ultrasonic wave to be applied to a deeper portion of the skin, an ultrasonic wave having a frequency of approximately 1 MHz (0.5 to 2 MHz) that is a frequency of a comparatively low band is generated by one of the ultrasonic transducers 20 while, with respect to the ultrasonic wave used for allowing the medicine 1 to penetrate the organism 2, an ultrasonic wave having a frequency of approximately 2 MHz or more that is a frequency of a comparatively high band is generated by the other ultrasonic vibrator 20; thus, the ultrasonic waves having these two frequencies may be combined with each other, and simultaneously applied to the skin surface 2a. Moreover, the above-mentioned ultrasonic percutaneous penetration device can be switched so as to apply two or more ultrasonic waves having different frequencies, and can also apply two or more ultrasonic waves having different frequencies alternately or in succession. Here, the ultrasonic wave having a frequency of a comparatively high band, used for allowing the medicine 1 to penetrate the organism 2, may be set to not more than 10 MHz.

Thus, two or more ultrasonic waves having different frequencies are generated, and by simultaneously applying these two ultrasonic waves to the skin surface 2a in combination, two or more different functions, exerted by two or more ultrasonic waves having different frequencies, are combined together, and applied to the organism 2 (in combination); thus, it becomes possible to improve the penetrating property of the medicine 1 to the organism 2.

Moreover, the present invention may be provided with at least one or more physical stimulatory functions selected from the group consisting of a physical stimulatory function for improving the penetrating effect of the medicine 1 to the organism 2, a physical stimulatory function for raising the comfortable feeling for the user and a physical stimulatory function for adding another action. For example, the physical stimulatory function for improving the penetrating effect of the medicine 1 to the organism 2 includes a function for applying a warming stimulation to the target portion of the organism 2 that is subjected to penetration of the medicine 1, and an ultrasonic percutaneous penetration device A is arranged so as to include a heat-generating tool (warming tool) that applies a warming stimulation to the target portion of the organism 2 by utilizing warm water, warm wind, steam, infrared radiation, far infrared radiation, high frequency and the like in its casing 10 and irradiation unit 4. The physical stimulatory function for raising the comfortable feeling for the user, for example, includes a function for applying massaging stimulation to the target portion of the organism 2 that is subjected to penetration of the medicine 1, and an ultrasonic percutaneous penetration device A is arranged so as to include a massaging tool that applies a massaging stimulation, such as massaging, pounding, rubbing and repeated actions of pressing and releasing, to the target portion of the organism 2, in its casing 10 and irradiation unit 4. Moreover, the physical stimulatory function for adding another action, for example, includes a function for applying a cell-activating stimulation to the target portion of the organism 2 that is subjected to penetration of the medicine 1, and an ultrasonic percutaneous penetration device A is arranged so as to include a cell-activating tool that applies a cell-activating stimulation to the target portion of the organism 2 by utilizing electric stimulation, photic stimulation laser and the like, in its casing 10 and irradiation unit 4.

Next, the following description discusses the penetration method of the medicine 1 of the present invention more specifically.

### (Specific Example 1)

An ultrasonic percutaneous penetration device A, as shown in Fig. 1, was used. With respect to a medicine 1, a whitening agent was used. With respect to the whitening agent, 1 cc (1 cm³) of gel-state aqueous solution to which carboxy methylcellulose (CMC) containing 3 % of vitamin C derivatives (magnesium ascorbyl phosphate) was added was used. Here, the gel-state aqueous solution to which carboxy methylcellulose (CMC) containing no vitamin C derivatives was added corresponds to an ultrasonic wave transmission medium 25.

The above-mentioned whitening agent was applied to a skin surface 2a, and ultrasonic waves were then applied to this skin surface 2a of an organism 2 from the irradiation unit 4 of the ultrasonic percutaneous device A through the whitening agent so that the medicine 1 was allowed to penetrate the skin (epidermis basal lamina). At this time, the irradiation conditions of the ultrasonic waves were set as follows: frequency: 5 MHz, irradiation power to the skin surface 2a: 0.35 W/cm², probe area of the irradiation unit 4 (area in which the irradiation unit 4 is made in contact with the skin surface 2a through the whitening agent): 4.52 cm², an output method for ultrasonic waves (power): continuous output (duty ratio: 100 %), and irradiation time: 5 minutes.

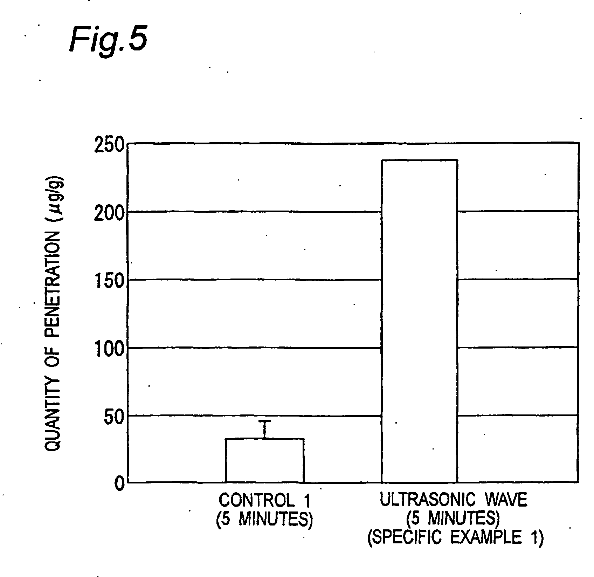

With respect to the above-mentioned specific example 1, the quantity of penetration of vitamin C (quantity of ascorbic acids per 1 g of skin) was measured. Moreover, for comparative purposes, with respect to those samples that had not been irradiated with ultrasonic waves (control 1) in the above-mentioned specific example 1, the quantity of penetration of vitamin C was also measured. Fig. 5 shows the results thereof.

As clearly shown by Fig. 5, specific example 1 had about 5 times as much as vitamin C penetration in comparison with control 1, thereby indicating that the application of ultrasonic waves provided high penetration effects for the medicine 1 and its active ingredient in comparison with control 1.

### (Specific Example 2)

First, as shown in Fig. 2, the ultrasonic wave transmission medium 25 was applied to a skin surface 2a in the same manner as described above, and ultrasonic waves were applied to the skin surface 2a by using the same ultrasonic percutaneous penetration device A as specific example 1 through the ultrasonic wave transmission medium 25, and allowed to penetrate the skin surface 2a. The same irradiation conditions as those of specific example 1 were used. Next, after the irradiation with ultrasonic waves, the ultrasonic wave transmission medium 25 was removed from the skin surface 2a, and this was allowed to stand still for 30 minutes. Thereafter, the same whitening agent as that of specific example 1 was applied to the skin surface 2a at the portion subjected to the irradiation with ultrasonic waves.

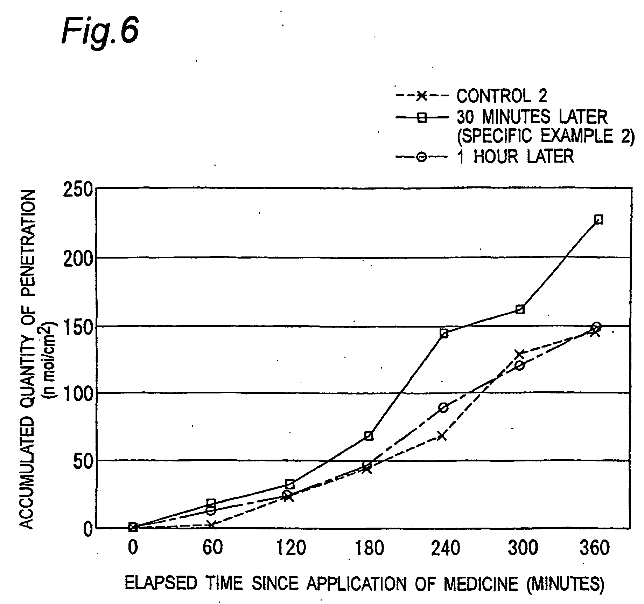

With respect to this specific example 2, the quantity of penetration of vitamin C (quantity of ascorbic acids per 1 g of skin) after the application of the whitening agent was measured on a time basis. Moreover, for comparative purposes, with respect to those samples that had not been irradiated with ultrasonic waves (control 2) in the above-mentioned specific example 2 and those samples to which the whitening agent was applied after a lapse of one hour from the irradiation of ultrasonic waves, the quantity of penetration of vitamin C was measured on a time basis. Fig. 6 shows the results thereof.

As clearly indicated by Fig. 6, specific example 2 had a greater accumulated quantity of penetration of vitamin C in comparison with control 2 and those samples to which the whitening agent was applied after a lapse of one hour since the irradiation with ultrasonic waves, and in comparison with control 2 and those samples to which the whitening agent was applied after a lapse of one hour since the irradiation with ultrasonic waves, specific example 2 had a greater quantity of penetration of vitamin C, and it was found that those samples in which the medicine 1 was applied to the skin surface 2a within 30 minutes after the application of ultrasonic waves had higher penetrating effects for the medicine 1 and its active ingredient.

### (Specific Example 3)

After skin lotion (medicine 1) containing 5 % of vitamin C derivatives (magnesium ascorbyl phosphate) had been applied to the entire portion of the face with cotton, ultrasonic waves were applied to only the half portion of the face through a gel-state ultrasonic wave transmission medium 25 without containing any whitening component in the same manner as described above for 10 minutes. At this time, the irradiation conditions of the ultrasonic waves were set as follows: frequency: 1 MHz, irradiation power to the skin surface 2a: 0.5 W/cm², probe area of the irradiation unit 4: 4.52 cm², and an output method for ultrasonic waves (power): continuous output (duty ratio: 100 %).

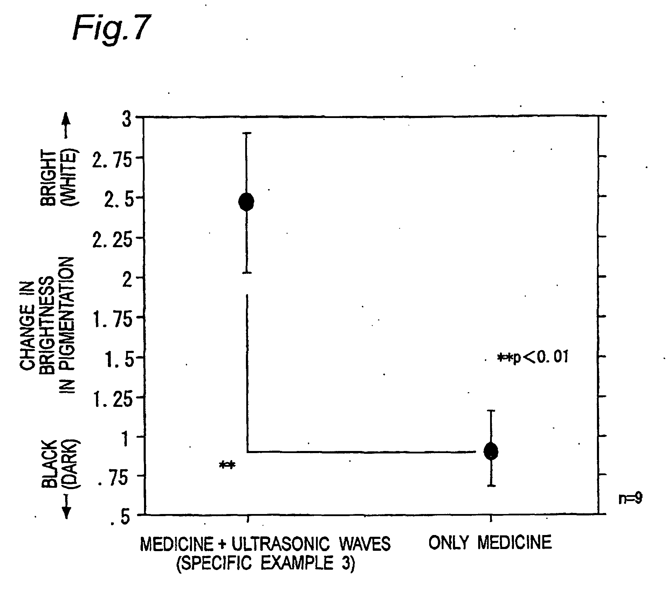

These operations were carried out almost every day, and the results obtained 2 to 3 months later are shown in Fig. 7. Fig. 7 compares the change in the color of pigmentation between the half of face (medicine + ultrasonic waves) to which both of the medicine 1 and ultrasonic waves were applied and the half of face (only medicine) to which only the medicine 1 was applied, and the results show that the color of pigmentation in the half of face to which both of the medicine 1 and ultrasonic waves were applied becomes brighter (whiter) in comparison with the half of face to which only the medicine 1 was applied, to show higher whitening effects. In other words, even when the medicine 1 was applied prior to the irradiation with ultrasonic waves, penetrating effects of the medicine 1 achieved by ultrasonic waves are sufficiently high.

## Claims

1. An ultrasonic percutaneous penetration device, which, upon allowing a medicine containing an active ingredient to penetrate an organism from a skin surface, allows vibration of ultrasonic waves to penetrate the organism from the skin surface, comprising:
an irradiation unit that applies ultrasonic waves having a frequency of not less than 0.5 MHz from skin or a surface capable of contacting the medicine; and
a control unit that controls irradiation conditions of the irradiation unit.

2. The ultrasonic percutaneous penetration device according to claim 1, wherein the control unit controls at least one of factors including the frequency, irradiation power, period between on and off of power and irradiation time, which are irradiation conditions of ultrasonic waves.

3. The ultrasonic percutaneous penetration device according to claim 1, further comprising: a detection unit that detects the depth of a portion for penetration of the medicine,
wherein the control unit controls the irradiation conditions so as to allow the medicine to penetrate to the depth detected by the detection unit.

4. The ultrasonic percutaneous penetration device according to claim 1, wherein the irradiation unit applies not less than two ultrasonic waves having different frequencies.

5. The ultrasonic percutaneous penetration device according to claim 4, wherein the irradiation unit applies an ultrasonic wave having a frequency of virtually 1 MHz and an ultrasonic wave having a frequency of not less than 2 MHz.

6. The ultrasonic percutaneous penetration device according to claim 1, further comprising: at least one tool selected from the group consisting of a thermal tool for warming a portion to be subjected to penetration of the medicine, a massaging tool for repeatedly pressing and releasing the portion to be subjected to penetration of the medicine, an electrostimulator that applies electrical stimulation to the portion to be subjected to penetration of the medicine and a photostimulator that applies photic stimulation to the portion to be subjected to penetration of the medicine.

7. An ultrasonic percutaneous penetration kit, which, upon allowing a medicine containing an active ingredient to penetrate an organism from a skin surface, allows vibration of ultrasonic waves to penetrate the organism from the skin surface, comprising:
a medicine containing an active ingredient;
an irradiation unit that applies ultrasonic waves having a frequency of not less than 0.5 MHz from a surface capable of contacting the medicine; and
a control unit that controls irradiation conditions of the irradiation unit.

8. The ultrasonic percutaneous penetration kit according to claim 7, wherein the control unit controls the frequency of the ultrasonic waves to a frequency within a range from 3 to 7 MHz.

9. The ultrasonic percutaneous penetration kit according to claim 8, wherein the active ingredient is at least one active ingredient selected from the group consisting of vitamin C, vitamin C derivatives, kojic acid, glucoside, glutathione, kiwifruit extract, rose fruit extract, arbutin and acerola extract.

10. The ultrasonic percutaneous penetration kit according to claim 7, wherein the active ingredient is at least one active ingredient selected from the group consisting of vitamin A, vitamin A acid derivatives, retinol, glutathione, α-hydroxy acid and a cell activation agent.

11. The ultrasonic percutaneous penetration kit according to claim 7, wherein: the active ingredient is at least one active ingredient selected from the group consisting of vitamin B group, capsaicin and caffeine, and the frequency of ultrasonic wave is controlled to not less than 0.7 MHz by the control unit.

12. The ultrasonic percutaneous penetration kit according to claim 7, wherein the active ingredient is at least one active ingredient selected from the group consisting of a thiocarbamate-based agent, an imidazole-based agent, an allylamine-based agent, an amorolfine-based agent, an undecylenic acid and derivatives thereof, an antifungal agent and an antitrichophyton agent

13. The ultrasonic percutaneous penetration kit according to claim 7, wherein the medicine is impregnated into a base material.

14. An ultrasonic percutaneous penetration method comprising the step of: simultaneously as a medicine containing an active ingredient is made in contact with the skin, applying ultrasonic waves having a frequency of not less than 0.5 MHz to a skin surface through the medicine.

15. An ultrasonic percutaneous penetration method comprising the step of: after a medicine containing an active ingredient has been made in contact with the skin, applying ultrasonic waves having a frequency of not less than 0.5 MHz to a skin surface through a medium that transmits ultrasonic waves.

16. An ultrasonic percutaneous penetration method comprising the step of: after having applied ultrasonic waves having a frequency of not less than 0.5 MHz to a skin surface, a medicine containing an active ingredient is made in contact with the skin to which the ultrasonic waves have penetrated.

17. An ultrasonic percutaneous penetration method comprising the steps of:
selecting two or more processes from the following three processes: a process in which a medicine containing an active ingredient is made in contact with the skin; a process in which ultrasonic waves having a frequency of not less than 0.5 MHz are applied to the skin surface; and a process in which, simultaneously as the medicine containing an active ingredient is made in contact with the skin, ultrasonic waves having a frequency of not less than 0.5 MHz are applied to the skin surface through the medicine, and
carrying out the selected processes time-serially in succession.
